# EUROPEAN PATENT APPLICATION

(11) **EP 4 321 092 A1**
(43) Date of publication of application: **14.02.2024**
(21) Application number: 22202827.6
(22) Date of filing: 20.10.2022
(51) Int. Cl.: A61B 5/11, A61B 5/00

(54) **FITNESS TRACKING DEVICE AND METHOD**

(30) Priority: 11.08.2022 KR 20220100670
(71) Applicant: Beflex Inc., Seoul 06628 (KR)
(72) Inventor: Choi, Hyeob, 34139 Yuseong-gu, Daejeon (KR); Jung, Chang Keun, 35214 Seo-gu, Daejeon (KR); Kim, Jin Kyuk, 34116 Yuseong-gu, Daejeon (KR)
(74) Representative: Gulde & Partner

(57) **Abstract**

The present invention relates to a fitness tracking method for workouts, including: a calibration step of setting local axes, based on the sensed data from a body sensor and an arm sensor; a workout readiness posture detection step of determining whether a workout readiness posture is taken, based on a first designated axis value for workout readiness posture detection; a workout parameter data derivation step of deriving workout parameter data, based on a second designated axis value for detecting the number of motions or whether a workout posture is maintained; and a workout end detection step of detecting the end of a workout, based on a third designated axis value for determining whether the corresponding workout is ended.

## Description

### BACKGROUND OF THE INVENTION

### Cross Reference to Related Application of the Invention

The present application claims the benefit of Korean Patent Application No. 10-2022-0100670 filed in the Korean Intellectual Property Office on August 11, 2022, the entire contents of which are incorporated herein by reference.

### Field of the Invention

The present invention relates to a fitness tracking device and method, and more specifically, to a measurement/analysis device and method for deriving workout parameters of motions of a variety of workouts from acceleration and angular velocity information sensed from a user's head and wrist.

### Background of the Related Art

Wearable devices become a big topic in a digital device market and are expected to provide many changes in people's daily lives. Among the wearable devices, fitness trackers have been prevailed so that they initially function to provide only the amount of activity of a user by means of an accelerometer sensor and are recently developed to collect the user's biological information by means of a variety of sensors. They are further improved so that they provide the user's desired information by using the data according to the characteristics of workouts. In this case, wearable fitness trackers having the form of being attached to the user's wrist become the center of the wearable device market because they are conveniently worn on the user.

However, the fitness trackers worn on the user's wrist are hard to sense various workout motions because degrees of freedom in the motions of the wrist are big. In specific, most of muscle-strengthening workouts are done to cause the movements of the user's body and arms, and in this case, it is difficult to accurately sense the movements only by using the sensor attached to his or her wrist. Therefore, there is a definite need to develop a new fitness tracker capable of being conveniently worn on the user and accurately sensing the movements occurring on his or her muscle-strengthening workouts. In this case, various workout motions can be measured and analyzed from the movements of the user's head and wrist because the movements of his or her body and arm are similar to the movements of his or her head and wrist, and further, the acceleration and angular velocity of the head and wrist can be measured by using wireless earphones and a smart watch that are popularized recently, so that they become very accessible workout information.

### SUMMARY OF THE INVENTION

Accordingly, the present invention has been made in view of the above-mentioned problems occurring in the related art, and it is an object of the present invention to provide a fitness tracking device and method that is capable of deriving the workout parameters of various workouts from the acceleration and angular velocity information of a user's head and wrist, thereby providing significant workout analysis information to people who do workouts and providing high accessibility thereto.

To accomplish the above-mentioned objects, according to the present invention, there is provided a fitness tracking method for workouts, including: a calibration step of setting local axes, based on the sensed data from a body sensor and an arm sensor; a workout readiness posture detection step of determining whether a workout readiness posture is taken, based on a first designated axis value for workout readiness posture detection; a workout parameter data derivation step of deriving workout parameter data, based on a second designated axis value for detecting the number of motions or whether a workout posture is maintained; and a workout end detection step of detecting the end of a workout, based on a third designated axis value for determining whether the corresponding workout is ended.

According to the present invention, desirably, the first to third designated axis values may include axis type information and value type information, the axis type information including information of local axes or global axes, information of any one of acceleration, angular velocity, and angle, and information of any one of x, y, z, roll, pitch, and yaw axes, and the value type information including information of any one of a sensed value, an average value, a previous sensed value, an angle value, a magnitude value, and an integrated value of any one of the sensed value, the average value, the previous sensed value, the angle value, and the magnitude value.

According to the present invention, desirably, the fitness tracking method may further include the step of selecting any one of the body sensor and the arm sensor and selecting the first to third designated axis values.

According to the present invention, desirably, the local axes may include x, y, and z axes of the body sensor produced based on looking ahead and behind data and looking up and down data which are produced when a guide screen appears on a display unit and on left and right direction data produced by externalizing the looking ahead and behind data and the looking up and down data.

According to the present invention, desirably, the local axes may further include x, y, and z axes of the arm sensor produced based on left and right data of the back of the hand and up and down data of the back of the hand which are produced when a guide screen appears on the display unit and on forward and backward data of the back of the hand produced by externalizing the left and right data of the back of the hand and the up and down data of the back of the hand.

According to the present invention, desirably, the data determining whether the workout posture is maintained may include workout velocity, information on whether correct postures are maintained, and information on whether the ranges of the motions are appropriate, which are determined according to the second designated axis value.

Other exemplary embodiments of the present invention are suggested in the description and drawings.

### BRIEF DESCRIPTION OF THE DRAWINGS

The above and other objects, features and advantages of the present invention will be apparent from the following detailed description of the embodiments of the invention in conjunction with the accompanying drawings, in which:
FIG. 1 is a schematic block diagram showing a configuration of a fitness tracking device according to the present invention;
FIG. 2 is an exemplary view showing a state where a body sensor and an arm sensor of the fitness tracking device according to the present invention are worn on a user's body and arm;
FIG. 3 is a flowchart showing a workout posture analysis method according to the present invention;
FIGs. 4 and 5 are exemplary views showing push-up workout analysis according to the present invention;
FIGs. 6 and 7 are exemplary views showing sit-up workout analysis according to the present invention;
FIGs. 8 and 9 are exemplary views showing squat workout analysis according to the present invention;
FIGs. 10 and 11 are exemplary views showing lunge workout analysis according to the present invention;
FIGs. 12 and 13 are exemplary views showing jump rope workout analysis according to the present invention;
FIGs. 14 and 15 are exemplary views showing stair climbing workout analysis according to the present invention; and
FIG. 16 is an exemplary view showing plank workout analysis according to the present invention.

### DETAILED DESCRIPTION OF THE PREFERRED EMBODIMENTS

The description as will be discussed below just suggests the principle of the present invention. It should be appreciated that various devices having the principle of the present invention and within the concept and range of the present invention may be invented by those skilled in the art even though they are not explained or shown in detail. Further, all of conditional terms and embodiments described in the present invention are obviously intended to allow the concept of the present invention to be understood.

It shall be understood that, although the terms "first," "second," "third," etc. may be used herein to describe various information, the information should not be limited to have main/sub or master/slave order by these terms.

Objects, characteristics and advantages of the present invention will be more clearly understood from the detailed description as will be described below and the attached drawings, and those skilled in the art will easily embody the technical scope of the present invention.

The features of various embodiments of the present invention may be partially or entirely combined with one another and technically operate cooperatively with one another, which will be appreciated by those skilled in the art. Further, the embodiments of the present invention may be embodied independently of one another or combinedly with one another.

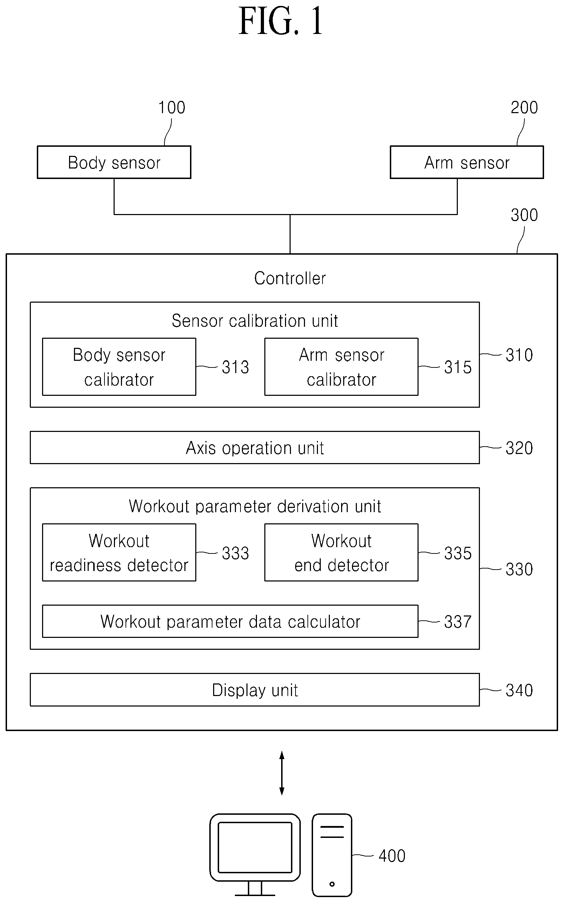

Now, an explanation of embodiments of the present invention will be given in detail with reference to the attached drawings. Referring first to FIG. 1, a fitness tracking device according to the present invention will be described.

As shown in FIG. 1, a fitness tracking device according to the present invention includes a body sensor 100, an arm sensor 200, and a controller 300. According to the present invention, in this case, the body sensor 100 and the arm sensor 200 include accelerometer sensors built in earphones or smart watches, and of course, they may include gyro sensors or gravity sensors.

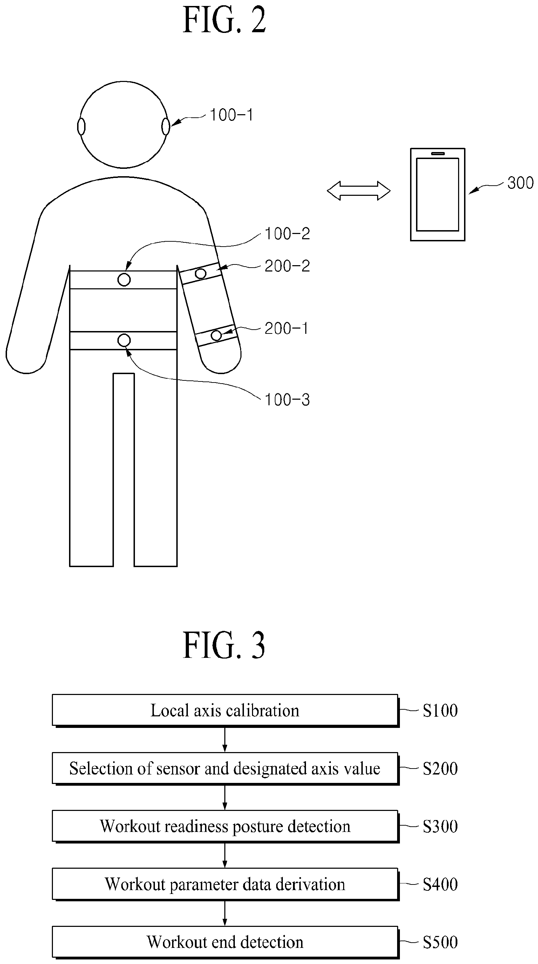

Referring to FIG. 2, the body sensor 100 is desirably an accelerometer sensor built in the earphones. However, the body sensor 100 may be attached to a user's body by means of a band or other devices worn on the user's ears or head, without being limited to the earphones. For example, the body sensor 100 may be attached to a breast band 100-2 or a waist band 100-3. The body sensor 100 may be attached to all of regions where the movements of the body occur representatively. Further, the body sensor 100 is accommodated in a separate housing from the controller 300, and otherwise, it may be accommodated in the same housing as the controller 300.

The arm sensor 200 is desirably an accelerometer sensor built in a smart watch 200-1 worn on the user's wrist. However, the arm sensor 200 may be attached to an arm band 200-2 or other arm regions where the movements of the arm occur representatively, without being limited thereto.

In the description, an example where the accelerometer sensor built in the earphones 100-1 is used as the body sensor 100 and the accelerometer sensor built in the smart watch 200-1 as the arm sensor 200 will be given below. According to the present invention, above all, the earphones and the smart watch can be worn easily on the user and their sensing positions can be appropriate, and accordingly, they are most desirable sensors. Of course, it is possible that the body sensor and the arm sensor may be worn on different positions of the user, as shown in FIG. 2.

The controller 300 automatically detects workout readiness or end and produces workout data, based on the sensed data from the body sensor 100 and the arm sensor 200. In specific, the controller 300 includes a sensor calibration unit 310 for calibrating the body sensor 100 and the arm sensor 200, an axis operation unit 320, and a workout parameter derivation unit 330. The controller 300 and the respective components of the controller 300 are application software, a software module, a hardware module, and a combination of software and hardware, which operate in a cellular phone, a tablet PC, a PC, and the like that wirelessly communicate with the body sensor 100 and the arm sensor 200.

In the case where a guide screen is provided on a display unit 340 of the controller 300 so that the user takes an action correspondingly to the guided screen, the sensor calibration unit 310 calibrates the workout direction of the user, based on the sensed data from the body sensor 100 and the arm sensor 200. A specific explanation of the calibration will be given later with reference to FIG. 3. In this case, the sensor calibration unit 310 includes a body sensor calibrator 313 and an arm sensor calibrator 315.

Further, the axis operation unit 320 operates local axis data and global axis data from the collected data from the sensors.

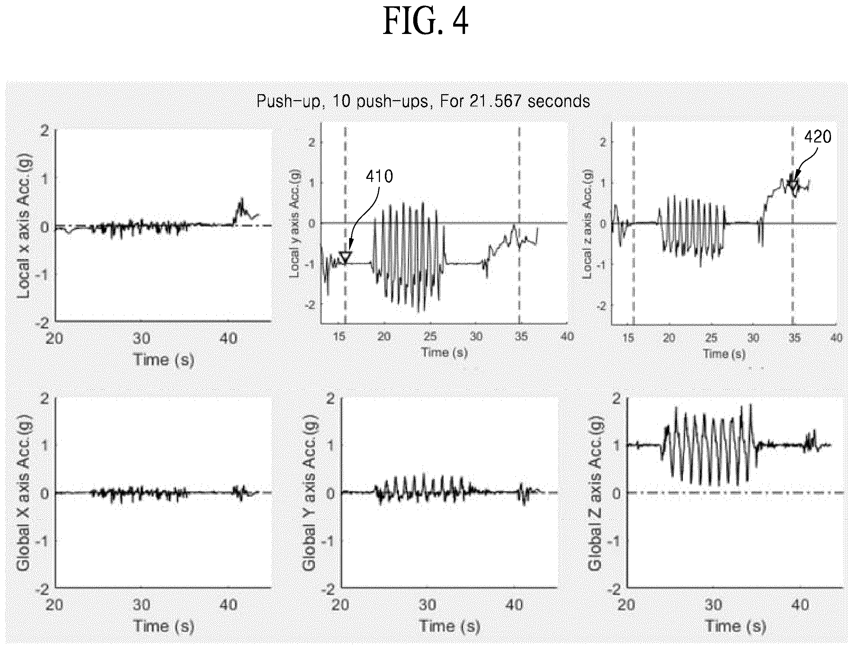

The workout parameter derivation unit 330 senses workout readiness postures and workout end according to types of workouts, sets and analyzes the section between workout readiness and end time points as a workout section, and derives workout parameter data. The workout parameter derivation methods are different according to the types of workouts, and they will be explained in detail with reference to FIGs. 4 to 16. In this case, the workout parameter derivation unit 330 provides an interface allowing the user to input the types of workouts to the display unit 740. Further, the workout parameter derivation unit 330 automatically discriminates the types of workouts according to the data derived from a workout readiness detector 333.

Further, the controller 300 communicates with a server 400, builds a database, and periodically updates the reference of the workout parameter derivation by workout according to the machine-learned contents of the database.

Now, an explanation of a fitness tracking method according to the present invention will be given in detail with reference to FIG. 3.

First, a fitness tracking method according to the present invention is performed by calibrating the sensed data with the local axis, based on the sensed data, at step S100. Fitness tracking sensors may be different in worn positions and directions because people have different physical shapes or skeletons, and even though they do the same workouts, deviations may be generated from measured workout information.

To remove such deviations, calibration is needed, and accordingly, if the guide screen is provided to the user so that he or she looks ahead (behind) and up (down), looking ahead and behind data and looking up and down data are generated using the value of the body sensor 100 generated while he or she is looking. The arm sensor 200 generates up and down data of the back of the hand and left and right data of the back of the hand with respect to the direction seen of the back of the hand. In this case, the looking ahead and behind data, the up and down data of the back of the hand, the looking up and down data, and the left and right data of the back of the hand are generated using only one direction of available two directions (e.g., forward and backward directions).

The looking ahead and behind data represent the sensed data of the body sensor 100 when the user looks ahead, and the looking up and down data represent the accelerometer data measured when he or she bows his or her head. The up and down data of the back of the hand represent the accelerometer data measured when the back of his or her hand looks up or down, and the left and right data of the back of the hand represent the accelerometer data measured when the back of his or her and looks left or right sides with respect to he or she who looks ahead.

The looking ahead and behind data and the looking up and down data are externalized to find the left and right directions of the body sensor 100, and in this case, x, y, and z axes of the body sensor 100 are set based on front and back, up and down, and left and right directions. In the same manner as above, x, y, and z axes of the arm sensor 200 are set. The set x, y, and z axes are local axis data.

The local axis of the local axis data is obtained by operating at least one of the directions (the body looking ahead and behind, the body looking up and down, the back of the hand looking up and down, and the back of the hand looking left and right) as defined above as an axis. In specific, the x, y, and z axes or roll, pitch, and yaw axes as the reference of the local axis data are determined based on the body looking ahead and behind, the body looking up and down, the back of the hand looking up and down, and the back of the hand looking left and right, and the axis operation unit 320 calibrates the sensed data with respect to the determined axes to produce the local axis data.

The global axis data represent data with respect to fixed axis directions, like a definition where gravitational acceleration is set with an up and down direction (as the z axis), a true north direction (or close to the front set on the local axis data and vertical with respect to the up and down direction) is set as the y axis, and a direction vertical with respect to the two directions as mentioned above is set as the x axis. In this case, any one axis of the global axis data includes gravitational acceleration. The local axis data and the global axis data are operated by the axis operation unit 320, based on the sensed data.

Further, the types of sensors and designated axis values are selected according to the types of workouts at step S200. However, the types of sensors and the designated axis values are selected before or after the calibration step. For example, if the types of workouts are automatically determined according to the axis directions upon the calibration, the types of sensors and the designated axis values are selected after the calibration, and contrarily, if the types of workouts are designated by the user, the selection of the types of sensors and the designated axis values are performed before the calibration. An explanation of the designated axis values will be given in detail later.

The body sensor 100, the arm sensor 200, or the combination of the two sensors is selected according to the types of workouts. In this case, the sensed data received from the selected sensor are calculated and produced as the local axis data and the global axis data by means of the axis operation unit 320. The workout parameter derivation unit 330 performs workout readiness detection, workout end detection, and workout parameter data derivation, based on the local axis data/global axis data produced from the axis operation unit 320.

After the sensor calibration and the sensor selection have been finished, further, the workout readiness detection is performed at step S300. At the workout readiness detection step S300, if the designated axis values of the designated sensor according to the types of workouts are within a first reference range for a given period of time, the workout readiness detector 333 determines that the readiness posture of the corresponding workout is taken. If necessary, additionally, if the designated axis values are within a second reference range, the workout readiness detector 333 determines that the readiness posture of the corresponding workout is taken. That is, the workout readiness detector 333 accurately determines that the readiness posture of the corresponding workout is taken by using the plurality of designated axis values simultaneously.

In this case, the designated axis values include axis type information, designated axis information, and value type information. The axis type information includes information of the local axes or the global axes, information of any one of acceleration, angular velocity, and angle, and information of any one of x, y, z, roll, pitch, and yaw axes. The value type information includes information of any one of a sensed value, an average value, a previous sensed value, an angle value, a magnitude value, and an integrated value of any one of the sensed value, the average value, the previous sensed value, the angle value, and the magnitude value. The previous sensed value represents the sensed value for a predetermined period of time before a detection time point or calculation time point, the average value represents the sensed value for the predetermined period of time, and the sensed value on the value type information represents the value derived at the detection time point or the calculation time point. Further, the magnitude value represents the magnitude value of a vector when the x, y, and z axes are defined.

In the description, further, the acceleration, velocity, and position are represented with units of g(9.81m/s2), g*s(9.81m/s), and g*s2(9.81m), and they will be described below with no units for the brevity of the description.

For example, the designated axis values may be the sensed value for two seconds of local y axis acceleration and the average value of global -x axis angular velocity.

If the workout readiness posture detection is finished, the workout parameter data is calculated by a workout parameter data calculator 337 of the workout parameter derivation unit 330 at step S400. The workout parameter data include the number of motions determined, based on the maximum value or minimum value of the designated axis values. Further, the workout parameter data include information on whether workout postures are maintained or not, and the information on whether workout postures are maintained includes the workout velocity, information on whether correct postures are maintained, and information on whether the ranges of the motions are appropriate, which are determined according to the designated axis values.

While the workout parameter data are calculated, a workout end detector 335 of the workout parameter derivation unit 330 continuously performs monitoring, and if a workout end motion is sensed, calculating the workout parameter data is stopped. That is, the workout end detector 335 detects the workout end posture by monitoring the sensed data for the designated axes at step S500. The muscle-strengthening workouts may be stopped for a given period of time, without being continuously done, and therefore, it is difficult to determine that the workouts are ended just because they are not continuously done for the given period of time. Further, the workout parameter data calculation may occur erroneously owing to the motions of the user after the workouts are ended, and accordingly, it is necessary to detect whether the workouts are ended or not.

The workout end detector 335 determines that the corresponding workout is ended if the designated axis values of the designated sensor according to the types of workouts are within the first reference range for the given period of time. If necessary, additionally, if the designated axis values are within the second reference range, the workout end detector 335 determines that the corresponding workout is ended.

In this case, if the designated axis values related to the number of motions, the workout velocity, the information on whether correct postures are maintained, and the ranges of motions are out of a third reference range, the workout parameter derivation unit 330 transmits warning through the display unit 340.

Hereinafter, embodiments of each individual workout will be explained with reference to FIGs. 4 to 16, based on the tests performed by the applicant of the invention. The reference ranges of the tests are determined based on the values obtained by a number of persons who do workouts.

### Push-up

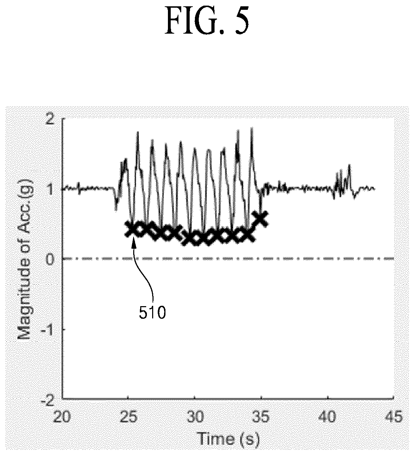

Referring to FIGs. 4 and 5, the workout readiness, workout end, and workout parameter of the push-up are defined as follows.

In the workout readiness of the push-up, the selected sensor is the accelerometer sensor of the body sensor 100, and the designated axis values are the sensed value for two seconds of local y axis acceleration and the average value of the local y axis acceleration. Referring to FIG. 4, in this case, the workout readiness detector 333 determines that the workout is ready at a time point 410 when the sensed value for two seconds of the local y axis acceleration is between -1.4 and -0.6 and the average value is between -1.2 and -0.8.

In specific, the first reference range is between -1.4 and -0.6, and the second reference range is between -1.2 and -0.8. According to the present invention, the first reference range and the second reference range are determined through the tests, but they may be updated through machine learning. In the case of the machine learning, an algorism such as support vector machine (SVM) is applied to the workout values of the professional of the corresponding workout to allow optimal designated axis value selection and reference ranges to be learned and updated.

According to another embodiment of the present invention, the selected sensor is the gyro sensor of the body sensor 100, and the designated axis value is an angular value for two seconds of the global -x axis acceleration. A time point when the angular value for two seconds of the global -x axis acceleration is maintained over 60° is determined as the workout readiness time point.

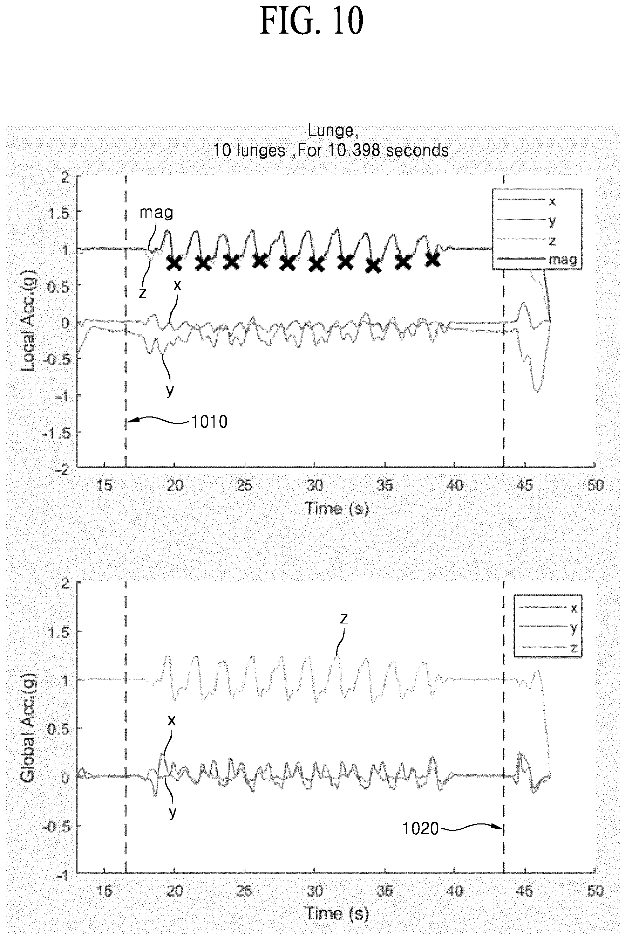

Further, the workout parameter data calculator 337 calculates the number of push-ups, and referring to FIG. 5, for example, the designated axis values in the workout parameter data calculation are the magnitude values of the local x, y, and z axis acceleration, and one push-up is sensed at a time point 510 when the magnitude values have minimum values less than 0.8 after having the maximum values greater than 1.3. In FIG. 5, 10 push-ups are measured.

According to another embodiment of the present invention, the designated axis value in the workout parameter data calculation is the sensed value of the local y axis acceleration, and one push-up is sensed at a time point when the sensed value has the maximum value greater than -0.8 after having the minimum value less than -1.3.

Otherwise, the designated axis value in the workout parameter data calculation is the sensed value of the global z axis acceleration, and one push-up is sensed at a time point when the sensed value has the minimum value less than 0.8 after having the maximum value greater than 1.3.

Further, the designated axis value in the workout parameter data calculation is the value obtained by subtracting 1 from the sensed value of the global z axis acceleration, the value type is an integrated value of the designated axis value two times from the workout readiness time point, and one push-up is sensed at a time point when the designated axis value has the maximum value greater than -0.01 after having the minimum value less than -0.01.

Meanwhile, the designated axis value of the end of the push-ups is the sensed value for three seconds of the local z axis acceleration, and in this case, the workout end detector 335 determines that the push-ups are ended at a time point 420 when the sensed value for three seconds of the local z axis acceleration is over 0.6. Further, the reference range of the workout end time point is determined through the machine learning, like the workout readiness time point.

According to another embodiment of the present invention, the selected sensor is the gyro sensor of the body sensor 100, and the designated axis value is an angular value for three seconds of the global -x axis acceleration, and a time point when the angular value for three seconds of the global -x axis acceleration is maintained at an angle less than 30° is determined as the workout end time point.

In the case of the push-up, further, the workout parameter data are data determining whether the body and the head are maintained in a straight line. In this case, the selected sensor for the workout parameter data is the gyro sensor, and the designated axis values are the sensed value of the local x axis and the sensed value (e.g., a front value when the user stands) of the global y axis. In the case where the sensed value of the local x axis and the sensed value of the global y axis have values of 45°, the workout parameter data determines that the body and the head are not maintained in a straight line. Otherwise, the selected sensor is the body sensor 100, and in this case, the sensor detects whether the sensed value of the local z axis acceleration is out of the reference range between -0.4 and 0.8.

In the push-ups, further, the workout parameter data are data determining whether excessive workout velocity is sensed. In this case, the selected sensor for the workout parameter data is the body sensor 100, and the designated axis value is the integrated value of the value obtained by subtracting 1 from the sensed value of the global z axis one time from the workout readiness time point. If the designated axis value is greater than 0.1, the workout parameter data determines that the workout velocity is excessively fast.

In the push-ups, moreover, the workout parameter data are data determining whether the ranges of motions are small. In this case, the selected sensor for the workout parameter data is the body sensor 100, and the designated axis value is the sensed value for five seconds of the integrated value of the value obtained by subtracting 1 from the sensed value of the global z axis two times from the workout readiness time point. If a difference between the maximum value and the minimum value of the designated axis value is less than 0.015, the workout parameter data determines that the ranges of motions are small.

### Sit-up

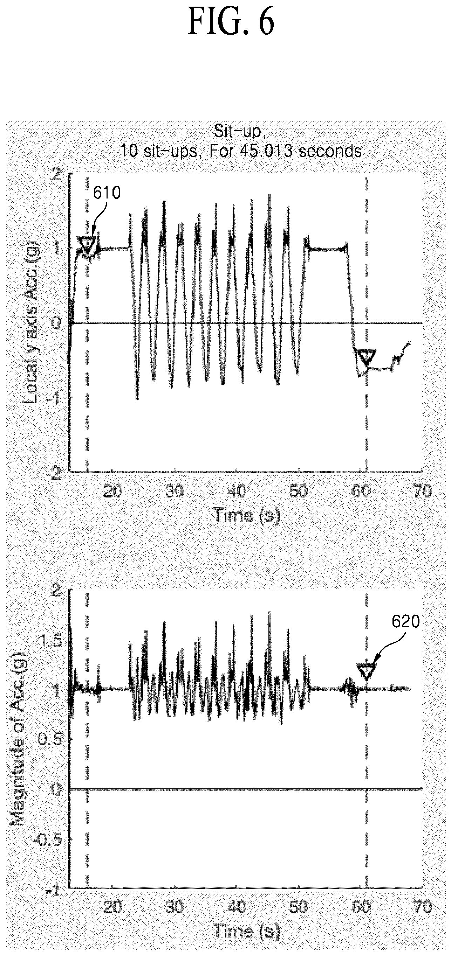

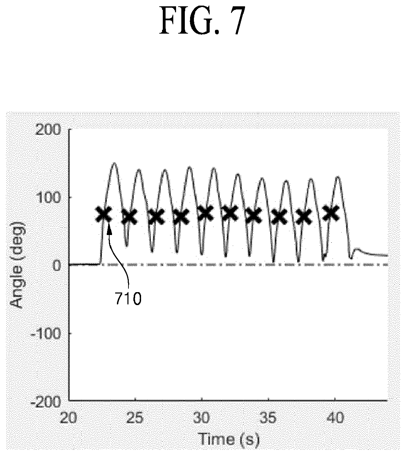

Referring to FIGs. 6 and 7, the workout readiness, workout end, and workout parameter of the sit-up are defined as follows.

In the workout readiness of the sit-up, the selected sensor is the body sensor 100, and the designated axis value is the sensed value for two seconds of the local y axis acceleration. Referring to FIG. 6, in this case, the workout readiness detector 333 determines that the workout is ready at a time point 610 when the sensed value for two seconds of the local y axis acceleration is between 0.8 and 1.2.

In specific, the first reference range is between 0.8 and 1.2. According to the present invention, the first reference range is determined through the tests, but it may be updated through machine learning, like the push-up.

According to another embodiment of the present invention, the selected sensor is the gyro sensor of the body sensor 100, and the designated axis value is an angular value for two seconds of the global x axis acceleration. A time point when the angular value for two seconds of the global x axis acceleration is maintained over 60° is determined as the workout readiness time point.

Further, the workout parameter data calculator 337 calculates the number of sit-ups (the number of motions), and the designated axis value in the workout parameter data calculation is the sensed value of the local y axis acceleration, and one sit-up is sensed at a time point when the sensed value has the minimum value less than 0.

According to another embodiment of the present invention, the designated axis value in the workout parameter data calculation is the sensed value of the global y axis acceleration, and one sit-up is sensed at a time point when the sensed value has the minimum value less than 0.

Otherwise, the selected sensor is the gyro sensor of the body sensor 100, and the designated axis value is an angular value of the global roll axis. One sit-up is sensed at a time point 710 when the angular value is maintained over 70°. In FIG. 7, 10 sit-ups are measured.

Meanwhile, the designated axis values of the end of the sit-ups are the magnitude values for three seconds of the local x, y and z axis acceleration and the sensed value of the local y axis acceleration, and in this case, the workout end detector 335 determines that the sit-ups are ended at a time point 620 when the magnitude values are between 0.8 and 1.2 and the sensed value is less than 0.6. Further, the reference range of the workout end time point is determined through the machine learning, like the workout readiness time point.

According to another embodiment of the present invention, the selected sensors are the accelerometer sensor and the gyro sensor of the body sensor 100, and the designated axis values are the magnitude values for three seconds of the local x, y and z axis acceleration and the angular value for three seconds of the global -x axis acceleration. The workout end detector 335 determines that the sit-ups are ended at a time point when the magnitude values are between 0.8 and 1.2 and the angular value is less than 30°.

### Squat

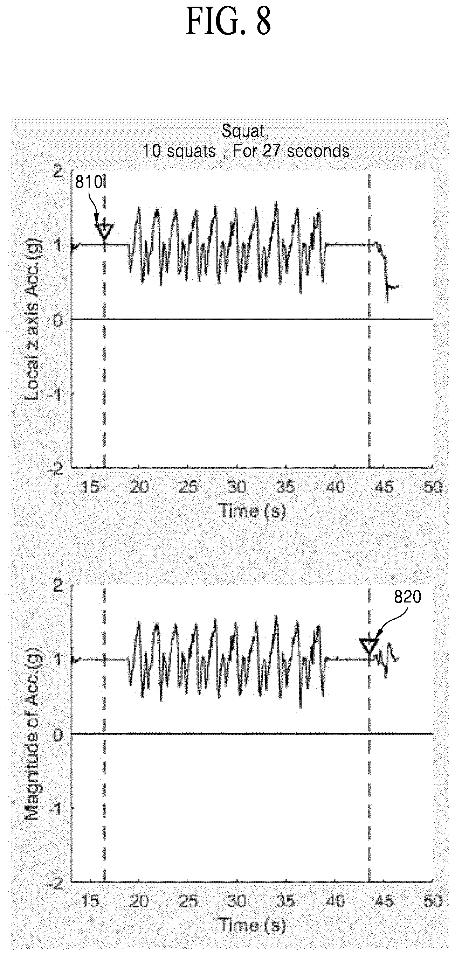

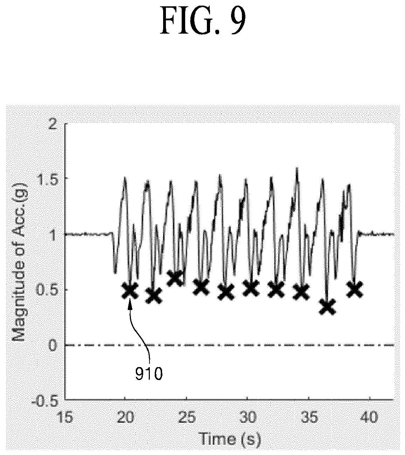

Referring to FIGs. 8 and 9, the workout readiness, workout end, and workout parameter of the squat are defined as follows.

In the workout readiness of the squat, the selected sensor is the body sensor 100, and the designated axis value is the sensed value for two seconds of the local z axis acceleration. Referring to FIG. 8, in this case, the workout readiness detector 333 determines that the workout is ready at a time point 810 when the sensed value for two seconds of the local z axis acceleration is between 0.8 and 1.2.

In specific, the first reference range is between 0.8 and 1.2. According to the present invention, the first reference range is determined through the tests, but it may be updated through machine learning.

Further, the workout parameter data calculator 337 calculates the number of squats, and referring to FIG. 9, for example, the designated axis values in the workout parameter data calculation are the magnitude values of the local x, y, and z axis acceleration, and one squat is sensed at a time point 910 when the magnitude values have the minimum values less than 0.8 after having the maximum values greater than 1.2. In FIG. 9, 10 squats are measured.

According to another embodiment of the present invention, the designated axis value in the workout parameter data calculation is the sensed value of the local z axis acceleration, and one squat is sensed at a time point when the sensed value has the minimum value less than 0.8 after having the maximum value greater than 1.2.

Otherwise, the designated axis value in the workout parameter data calculation is the sensed value of the global z axis acceleration, and one squat is sensed at a time point when the sensed value has the minimum value less than 0.8 after having the maximum value greater than 1.2.

Further, the designated axis value in the workout parameter data calculation is the value obtained by subtracting 1 from the sensed value of the global z axis acceleration, the value type is an integrated value of the designated axis value two times from the workout readiness time point, and one squat is sensed at a time point when the designated axis value has the maximum value greater than -0.01 after having the minimum value less than -0.02.

Meanwhile, the designated axis values of the end of the squats are the magnitude values for three seconds of the local x, y and z axis acceleration, and in this case, the workout end detector 335 determines that the squats are ended at a time point when the magnitude values are between 0.8 and 1.2. Further, the reference range of the workout end time point is determined through the machine learning, like the workout readiness time point.

In the case of the squat, further, the workout parameter data are data determining whether the body correctly stands. In this case, the selected sensor for the workout parameter data is the body sensor 100, and the designated axis value is the sensed value of the local x axis acceleration or the sensed value of the global z axis acceleration. In the case where the sensed value has a peak value greater than 0.9 between the maximum values, the workout parameter data determines that the body correctly stands. Otherwise, the selected sensor is the body sensor 100, and the designated axis value is the value before and after the maximum value (between the neighboring maximum values) of the integrated value of the value obtained by subtracting 1 from the sensed value of the global z axis acceleration two times from the workout readiness time point. In this case, if there is a period greater than two seconds that satisfies the designated axis value greater than -0.03, the workout parameter data determines that the body correctly stands.

In the squats, further, the workout parameter data are data determining whether the center of gravity moves in forward and backward directions. In this case, the selected sensor for the workout parameter data is the body sensor 100, and the designated axis value is the absolute value of the integrated value of the sensed value of the global y axis acceleration two times from the workout readiness time point. If the absolute value is greater than 0.05, the workout parameter data determines that the center of gravity moves in forward and backward directions. Otherwise, the selected sensor for the workout parameter data is the body sensor 100, and the designated axis value is the absolute value of the integrated value of the sensed value of the global y axis acceleration one time from the workout readiness time point. In this case, if the absolute value is greater than 0.2, the workout parameter data determines that the center of gravity moves in forward and backward directions. Moreover, the selected sensor for the workout parameter data is the body sensor 100, and the designated axis value is the absolute value of the sensed value of the global y axis acceleration. In this case, if the absolute value is greater than 1, the workout parameter data determines that the center of gravity moves in forward and backward directions.

In the squats, moreover, the workout parameter data are data determining whether the ranges of motions are small. In this case, the selected sensor for the workout parameter data is the body sensor 100, and the designated axis value is the sensed value for five seconds of the integrated value of the value obtained by subtracting 1 from the sensed value of the global z axis acceleration two times from the workout readiness time point. If a difference between the maximum value and the minimum value of the designated axis value is less than 1/6 of the user's body height, the workout parameter data determines that the ranges of motions are small.

### Lunge

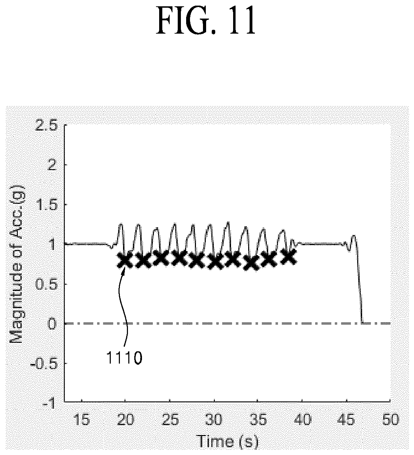

Referring to FIGs. 10 and 11, the workout readiness, workout end, and workout parameter of the lunge are defined as follows. However, the lunge workout utilizes the data processed by a low pass filter.

In the workout readiness of the lunge, the selected sensor is the body sensor 100, and the designated axis value is the sensed value for two seconds of the local z axis acceleration. Referring to FIG. 10, in this case, the workout readiness detector 333 determines that the workout is ready at a time point 1010 when the sensed value for two seconds of the local z axis acceleration is between 0.8 and 1.2.

In specific, the first reference range is between 0.8 and 1.2. According to the present invention, the first reference range is determined through the tests, but it may be updated through machine learning.

Further, the workout parameter data calculator 337 calculates the number of lunges, and referring to FIG. 11, for example, the designated axis values in the workout parameter data calculation are the magnitude values of the local x, y, and z axis acceleration, and one lunge is sensed at a time point 1110 when the magnitude values have the minimum values less than 1 after having the maximum values greater than 1.1. In FIG. 11, 10 lunges are measured.

According to another embodiment of the present invention, the designated axis value in the workout parameter data calculation is the sensed value of the local z axis acceleration, and one lunge is sensed at a time point when the sensed value has the minimum value less than 1 after having the maximum value greater than 1.1.

Otherwise, the designated axis value in the workout parameter data calculation is the sensed value of the global z axis acceleration, and one lunge is sensed at a time point when the sensed value has the minimum value less than 1 after having the maximum value greater than 1.1.

Further, the designated axis value in the workout parameter data calculation is the value obtained by subtracting 1 from the sensed value of the global z axis acceleration, the value type is an integrated value of the designated axis value two times from the workout readiness time point, and one lunge is sensed at a time point when the designated axis value has the maximum value greater than -0.01 after having the minimum value less than -0.02.

Meanwhile, the designated axis values of the end of the squats are the magnitude values for three seconds of local x, y and z axis acceleration, and in this case, the workout end detector 335 determines that the lunges are ended at a time point when the magnitude values are between 0.9 and 1.1. Further, the reference range of the workout end time point is determined through the machine learning, like the workout readiness time point.

In the case of the lunge, further, the workout parameter data are data determining whether the center of gravity moves in left and right directions. In this case, the selected sensor for the workout parameter data is the body sensor 100, and the designated axis value is the absolute value of the integrated value of the sensed value of the global x axis acceleration two times from the workout readiness time point. If the absolute value is greater than 0.05, the workout parameter data determines that the center of gravity moves in left and right directions. Otherwise, the selected sensor for the workout parameter data is the body sensor 100, and the designated axis value is the absolute value of the integrated value of the sensed value of the global x axis acceleration two times from the workout readiness time point. If the absolute value is greater than 0.2, the workout parameter data determines that the center of gravity moves in left and right directions. Moreover, if the absolute value of the sensed value of the global x axis acceleration is greater than 0.5, the workout parameter data determines that the center of gravity moves in left and right directions.

In the lunges, moreover, the workout parameter data are data determining whether the ranges of motions are small. In this case, the selected sensor for the workout parameter data is the body sensor 100, and the designated axis value is the sensed value for five seconds of the integrated value of the value obtained by subtracting 1 from the sensed value of the global z axis acceleration two times from the workout readiness time point. If a difference between the maximum value and the minimum value of the designated axis value is less than 1/6 of the user's body height, the workout parameter data determines that the ranges of motions are small.

### Jump rope

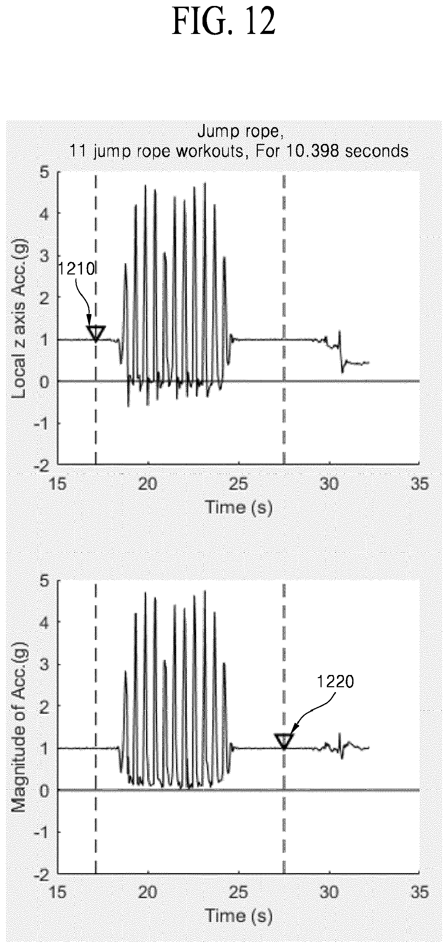

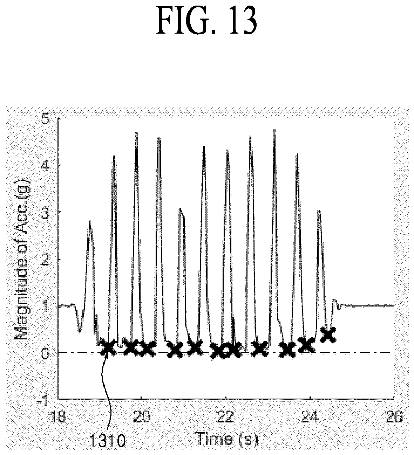

Referring to FIGs. 12 and 13, the workout readiness, workout end, and workout parameter of the jump rope are defined as follows.

In the workout readiness of the jump rope, the selected sensor is the body sensor 100, and the designated axis value is the sensed value for two seconds of the local z axis acceleration. Referring to FIG. 12, in this case, the workout readiness detector 333 determines that the workout is ready at a time point 1210 when the sensed value for two seconds of the local z axis acceleration is between 0.8 and 1.2.

In specific, the first reference range is between 0.8 and 1.2. According to the present invention, the first reference range is determined through the tests, but it may be updated through machine learning.

Further, the workout parameter data calculator 337 calculates the number of jump rope workouts, and referring to FIG. 13, for example, the designated axis values in the workout parameter data calculation are the magnitude values of the local x, y, and z axis acceleration, and one jump rope is sensed at a time point 1310 when the magnitude values have the minimum values less than 0.6 after having the maximum values greater than 1.5. In FIG. 13, 10 jump rope workouts are measured.

According to another embodiment of the present invention, the designated axis value in the workout parameter data calculation is the sensed value of the local z axis acceleration, and one jump rope workout is sensed at a time point when the sensed value has the minimum value less than 0.6 after having the maximum value greater than 1.5.

Otherwise, the designated axis value in the workout parameter data calculation is the sensed value of the global z axis acceleration, and one jump rope workout is sensed at a time point when the sensed value has the minimum value less than 0.6 after having the maximum value greater than 1.5.

Further, the designated axis value in the workout parameter data calculation is the value obtained by subtracting 1 from the sensed value of the global z axis acceleration, the value type is an integrated value of the designated axis value two times from the workout readiness time point, and one jump rope workout is sensed at a time point when the designated axis value has the maximum value greater than 0.005.

Meanwhile, the designated axis values of the end of the jump rope workouts are the magnitude values for three seconds of local x, y and z axis acceleration, and in this case, the workout end detector 335 determines that the jump rope workouts are ended at a time point when the magnitude values are between 0.8 and 1.2. Further, the reference range of the workout end time point is determined through the machine learning, like the workout readiness time point.

### Stair climbing

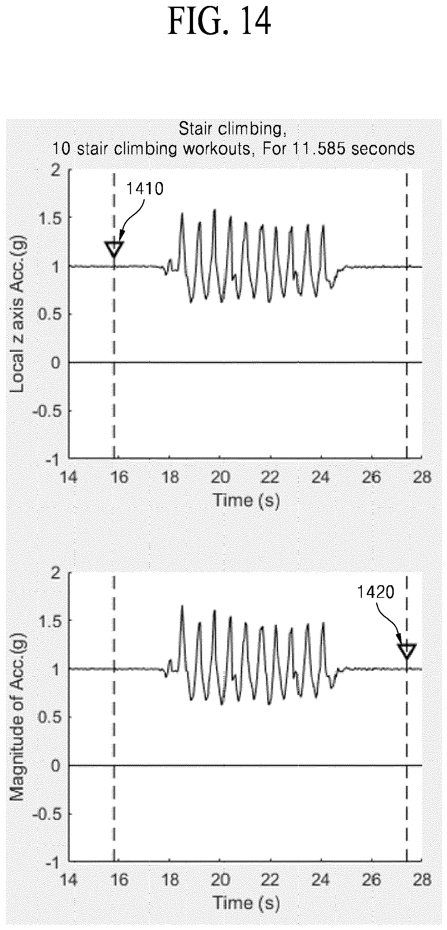

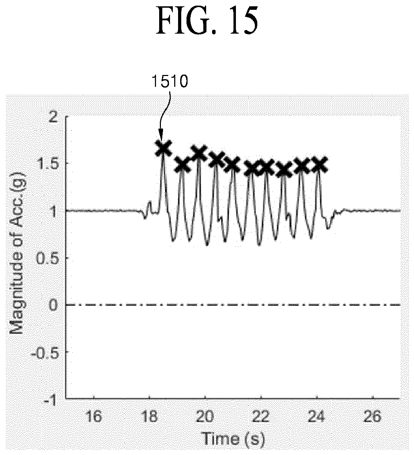

Referring to FIGs. 14 and 15, the workout readiness, workout end, and workout parameter of the stair climbing are defined as follows.

In the workout readiness of the stair climbing, the selected sensor is the body sensor 100, and the designated axis value is the sensed value for two seconds of the local z axis acceleration. Referring to FIG. 14, in this case, the workout readiness detector 333 determines that the workout is ready at a time point 1410 when the sensed value for two seconds of the local z axis acceleration is between 0.85 and 1.2.

In specific, the first reference range is between 0.85 and 1.2. According to the present invention, the first reference range is determined through the tests, but it may be updated through machine learning.

Further, the workout parameter data calculator 337 calculates the number of stair climbing workouts, and referring to FIG. 15, for example, the designated axis values in the workout parameter data calculation are the magnitude values of the local x, y, and z axis acceleration, and one stair climbing workout is sensed at a time point 1510 when the magnitude values have the maximum values greater than 1.2. In FIG. 15, 10 stair climbing workouts are measured.

According to another embodiment of the present invention, the designated axis value in the workout parameter data calculation is the sensed value of the local z axis acceleration, and one stair climbing workout is sensed at a time point when the sensed value has the maximum value greater than 1.2.

Otherwise, the designated axis value in the workout parameter data calculation is the sensed value of the global z axis acceleration, and one stair climbing workout is sensed at a time point when the sensed value has the maximum value greater than 1.2.

Meanwhile, the designated axis values of the end of the stair climbing workouts are the magnitude values for three seconds of the local x, y and z axis acceleration, and in this case, the workout end detector 335 determines that the stair climbing workouts are ended at a time point when the magnitude values are between 0.85 and 1.2. Further, the reference range of the workout end time point is determined through the machine learning, like the workout readiness time point.

In the case of the stair climbing workouts, further, the workout parameter data are data of increased heights. In this case, the selected sensor for the workout parameter data is the body sensor 100, and the designated axis value is the integrated value of the sensed value of the global z axis acceleration two times from the workout readiness time point.

In the case of the stair climbing workouts, moreover, the workout parameter data are data determining whether the user's head and body are inclined forward. In this case, the selected sensor for the workout parameter data is the gyro sensor of the body sensor 100, and the designated axis value is the angle between the orthogonal projection onto the global y and z axis plane as the sensed value of the global z axis acceleration and the global z axis. If the angle is greater than 45°, the workout parameter data determines that the user's head and body are inclined forward. Otherwise, the designated axis value is the sensed value of the local y axis acceleration, and if the sensed value is less than -0.8, the workout parameter data determines that the user's head and body are inclined forward.

### Plank

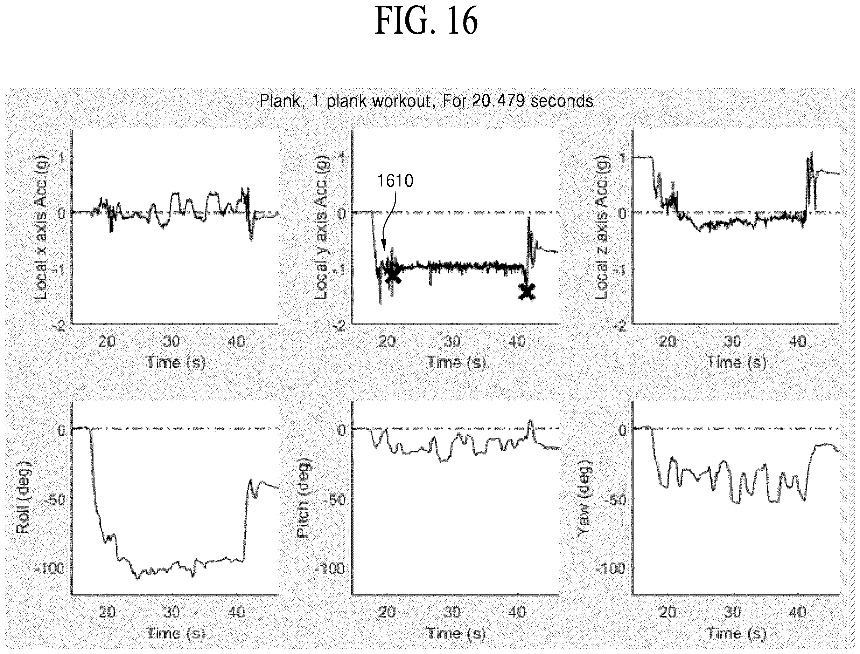

Referring to FIG. 16, the workout readiness, workout end, and workout parameter of the plank are defined as follows.

In the workout readiness of the plank, the selected sensor is the body sensor 100, and the designated axis values are the average value for two seconds of the local y axis acceleration and the magnitude values of the local axis acceleration. Referring to FIG. 16, in this case, the workout readiness detector 333 determines that the workout is ready at a time point 1610 when the average value for two seconds of the local y axis acceleration is less than -0.8 and the magnitude values of the local axis acceleration are between 0.7 and 1.3.

In specific, the first reference range is less than -0.8, and the second reference range is between 0.7 and 1.3. According to the present invention, the first reference range and the second reference range are determined through the tests, but they may be updated through machine learning.

Further, the workout parameter data calculator 337 calculates a plank time, and the plank time is the time from the time point when the plank starts to the time point when the plank is ended.

The designated axis values of the end of the plank workouts are the magnitude values of the local x, y and z axis acceleration and the sensed value of the local y axis acceleration, and in this case, the workout end detector 335 determines that the stair climbing workouts are ended at a time point when the magnitude values are less than 0.5 or greater than 1 or the sensed value of the local y axis acceleration is greater than -0.2.

In the case of the plank workouts, further, the workout parameter data are data determining whether fatigue rises during the workout. In this case, the selected sensor for the workout parameter data is the body sensor 100, and the designated axis value is the sensed value for three seconds of the local axis acceleration. If the standard deviation of each component is greater than a reference deviation, it is determined that the fatigue rises.

In the case of the plank workouts, moreover, the workout parameter data are data determining whether the user's head and body are maintained in a straight line. In this case, the selected sensor for the workout parameter data is the gyro sensor of the body sensor 100, and the designated axis value is the angle between the orthogonal projection onto the global y and z axis plane as the sensed value of the global z axis acceleration and the global z axis. If the angle is greater than 45°, the workout parameter data determines that the user's head and body are not maintained in a straight line. Otherwise, the designated axis value is the sensed value of the local z axis acceleration, and if the sensed value is out of the range of -0.4 and 0.8, the workout parameter data determines that the user's head and body are not maintained in a straight line.

### Dumbbell curl (Biceps workout)

Referring to FIG. 16, the workout readiness, workout end, and workout parameter of the dumbbell curl are defined as follows.

In the workout readiness of the dumbbell curl, the selected sensor is the arm sensor 200, and the designated axis values are the sensed value of the local y axis acceleration and the magnitude values for two seconds of the local axis acceleration. In this case, the workout readiness detector 333 determines that the workout is ready at a time point when the sensed value of the local y axis acceleration is between -0.4 and 0.4 and the magnitude values for two seconds of the local axis acceleration are between 0.8 and 1.2.

Further, the workout parameter data calculator 337 calculates the number of dumbbell curl workouts, and the designated axis values in the workout parameter data calculation are the magnitude values of the local x, y, and z axis acceleration, and one dumbbell curl workout is sensed at a time point when the magnitude values have the maximum values greater than 0.5.

According to another embodiment of the present invention, one dumbbell curl workout is sensed at a time point when the integrated value of the value obtained by subtracting 1 from the sensed value of the global z axis acceleration two times from the workout readiness time point has the maximum value greater than 0.01.

Otherwise, the selected sensor for the workout parameter data is the gyro sensor of the arm sensor 200, and the designated axis value in the workout parameter data calculation is the absolute value of the angle value of the local pitch (y) axis rotation. In this case, one dumbbell curl workout is sensed at a time point when the absolute value is greater than 80° in an absolute value increasing direction from the workout readiness time point.

According to the present invention, the fitness tracking device is configured to sense various workout motions from the acceleration information measured through the body sensor and the arm sensor, while being conveniently worn on the user's body and arm so that he or she can focus on the workouts, without having uncomfortable feelings.

According to the present invention, further, the fitness tracking device is configured to automatically sense the readiness and end of the workouts that are hard to be provided accurately by the existing arm sensor (smart watch and the like).

According to the present invention, moreover, the fitness tracking device is configured to automatically store workout data, thereby accurately measuring the muscle-strengthening workouts of the user.

The present invention may be modified in various ways and may have several exemplary embodiments. Accordingly, it should be understood that the invention covers all the modifications, equivalents, and replacements within the idea and technical scope of the invention. Therefore, the present invention is not to be restricted by the embodiments as mentioned above. It is therefore intended that the scope of the invention be limited not by this detailed description, but rather by the claims appended hereto.

## Claims

1. A fitness tracking method for workouts, comprising:
a calibration step of setting local axes, based on the sensed data from a body sensor and an arm sensor;
a workout readiness posture detection step of determining whether a workout readiness posture is taken, based on a first designated axis value for workout readiness posture detection;
a workout parameter data derivation step of deriving workout parameter data, based on a second designated axis value for detecting the number of motions or whether a workout posture is maintained; and
a workout end detection step of detecting the end of a workout, based on a third designated axis value for determining whether the corresponding workout is ended.

2. The fitness tracking method according to claim 1, wherein the first to third designated axis values comprise axis type information and value type information, the axis type information comprising information of local axes or global axes, information of any one of acceleration, angular velocity, and angle, and information of any one of x, y, z, roll, pitch, and yaw axes, and the value type information comprising information of any one of a sensed value, an average value, a previous sensed value, an angle value, a magnitude value, and an integrated value of any one of the sensed value, the average value, the previous sensed value, the angle value, and the magnitude value.

3. The fitness tracking method according to claim 1, further comprising the step of selecting any one of the body sensor and the arm sensor and selecting the first to third designated axis values.

4. The fitness tracking method according to claim 1, wherein the local axes comprise x, y, and z axes of the body sensor produced based on looking ahead and behind data and looking up and down data which are produced when a guide screen appears on a display unit and on left and right direction data produced by externalizing the looking ahead and behind data and the looking up and down data.

5. The fitness tracking method according to claim 4, wherein the local axes further comprise x, y, and z axes of the arm sensor produced based on left and right data of the back of the hand and up and down data of the back of the hand which are produced when a guide screen appears on the display unit and on forward and backward data of the back of the hand produced by externalizing the left and right data of the back of the hand and the up and down data of the back of the hand.

6. The fitness tracking method according to claim 1, wherein the data determining whether the workout posture is maintained comprises workout velocity, information on whether correct postures are maintained, and information on whether the ranges of the motions are appropriate, which are determined according to the second designated axis value.

7. The fitness tracking method according to claim 1, wherein if the first designated value includes the sensed value for two seconds of the local y axis acceleration and the average value of the local y axis acceleration, it is determined that a push-up workout is ready at a time point when the sensed value for two seconds of the local y axis acceleration is between -1.4 and -0.6 and the average value of the local y axis acceleration is between -1.2 and -0.8.
